# EUROPÄISCHE PATENTANMELDUNG

(11) **EP 1 066 841 A2**
(43) Veröffentlichungstag der Anmeldung: **10.01.2001**
(21) Anmeldenummer: 00101842.3
(22) Anmeldetag: 29.01.2000
(51) Int. Cl.: A61M 1/14

(54) **Einrichtung zur Versorgung von Dialysegeräten mit aufbereitetem Wasser**

(30) Priorität: 07.07.1999 DE 19931304
(71) Anmelder: KFH Kuratorium für Dialyse und Nierentransplationen e.V., 63263 Neu-Isenburg (DE)
(72) Erfinder: Plenzig, Steffen, 63263 neu-Isenburg (DE); Kaupa, Paul, 64331 Weiterstadt (DE); Kurina, Hans, 48268 Greven (DE)
(74) Vertreter: Flosdorff, Jürgen, Dr.

(57) **Zusammenfassung**

Die Einrichtung zur Versorgung von Dialysegeräten mit aufbereitetem Wasser enthält eine primäre Ringleitung, von der jeweils eine Zuleitung zu einem Wassereinlaßventil eines Dialysegerätes führt. Diese Zuleitung ist als eine sekundäre Ringleitung ausgebildet mit einem ersten Leitungsabschnitt, der von der primären Ringleitung zu dem Einlaßventil führt, und mit einem zweiten Leitungsabschnitt, der zur primären Ringleitung zurückführt und mit einer Engstelle versehen ist. Die sekundäre Ringleitung wird während und/oder außerhalb des Betriebs des Dialysegerätes von dem aufbereiteten Wasser durchströmt, wodurch die Ablagerung von Bakterien etc. in diesem Bereich verhindert ist. Außerdem kann die sekundäre Ringleitung in die Desinfektion der primären Ringleitung einbezogen werden.

## Beschreibung

Die Erfindung betrifft eine Einrichtung zur Versorgung von Dialysegeräten mit aufbereitetem Wasser, mit einer Ringleitung, von der jeweils eine Zuleitung zu einem Wassereinlaßventil eines Dialysegerätes führt.

Bisher werden die Dialysegeräte einer Dialysestation über Stichleitungen zwischen einer Ringleitung und den Dialysegeräten mit aufbereitetem Wasser versorgt. Diese Ausbildung hat den Nachteil, daß der Bereich der Wasserzuführung nicht in den Spül- bzw. Desinfektionskreislauf eingebunden ist. Die Ringleitung und das Dialysegerät können separat desinfiziert werden, was für die Zuleitung von der Ringleitung zum Dialysegerät jedoch bisher nicht möglich ist. Es ist bekannt, daß dieser Bereich Verursacher von mikrobiologischen Problemen im Gesamtsystem ist.

Der vorliegenden Erfindung liegt die Aufgabe zugrunde, den Bereich der Zuleitung von aufbereitetem Wasser zu dem Dialysegerät so auszubilden, daß dieser regelmäßig in die Spülprozesse eingebunden ist. Außerdem soll erreicht werden, daß das komplette System einer Desinfektion unterzogen werden kann.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, daß die Zuleitung zu dem Dialysegerät Bestandteil einer weiteren, sekundären Ringleitung ist, die während und/oder außerhalb des Betriebs des Dialysegerätes von dem aufbereiteten Wasser durchströmbar ist. Hierdurch können die Zuleitung sowie Teile des Dialysegerätes regelmäßig und dauerhaft durchströmt werden, wodurch die Gefahr der Ausbildung und Ansammlung organischer Ablagerungen wie Bakterien und deren Ausscheidungsprodukte in diesem Bereich weitestgehend beseitigt ist. Außerdem kann die sekundäre Ringleitung gemeinsam mit der primären Ringleitung desinfiziert werden, so daß sich mikrobiologische Probleme zuverlässig vermeiden lassen.

In weiterer Ausgestaltung der Erfindung ist vorgesehen, daß ein weiterer Leitungsabschnitt der sekundären Ringleitung zu der primären Ringleitung zurückführt, obwohl es grundsätzlich auch im Rahmen der Erfindung liegt, daß durch den weiteren Leitungsabschnitt das beim Spülvorgang durchströmende Wasser aus dem Wasserversorgungssystem abgeführt wird, d.h. nicht in die primäre Ringleitung zurückfließt. Wegen des hiermit verbundenen Wasserverbrauchs dürfte das Zurückführen in die primäre Ringleitung zu bevorzugen sein.

Weiter wird mit großem Vorteil vorgeschlagen, daß der weitere Leitungsabschnitt eine Engstelle hat. Diese Engstelle kann durch eine Drosseleinrichtung gebildet sein, die eine konstante Durchflußöffnung hat und eine vorbestimmte Wassermenge durchfließen läßt. Die Drosseleinrichtung kann aber alternativ hierzu auch verstellbar sein, so daß die die sekundäre Ringleitung beim Spülvorgang durchfließende Wassermenge einstellbar ist.

Durch die Engstelle wird erreicht, daß ein gegenüber dem die Zuleitung zu dem Wassereinlaßventil bildenden ersten Leitungsabschnitt verringerter Durchfluß durch den weiteren Leitungsabschnitt erfolgt. Dies bedeutet, daß das Dialysegerät durch den die Zuleitung bildenden ersten Leitungsabschnitt mit der erforderlichen Wassermenge versorgt wird und daß beim Spülen der sekundären Ringleitung während und/oder außerhalb des Betriebs des Dialysegerätes eine demgegenüber reduzierte Wassermenge durch diese sekundäre Ringleitung strömt.

Hierzu muß in dem weiteren Leitungsabschnitt nicht unbedingt eine örtlich begrenzte Engstelle in Form einer Drosseleinrichtung oder dergleichen vorgesehen sein, sondern es liegt im Rahmen der Erfindung, daß der weitere Leitungsabschnitt insgesamt einen kleineren freien Innendurchmesser als der erste Leitungsabschnitt haben kann.

Nach einem weiteren Vorschlag der Erfindung kann der weitere Leitungsabschnitt direkt an das Wassereinlaßventil des Dialysegerätes angeschlossen sein, das in diesem Fall bevorzugt durch ein Drei-Wege-Ventil oder ein Zwei-Wege-Ventil mit integriertem Überströmungskanal gebildet ist. Dabei kann das Drei-Wege-Ventil so eingestellt werden, daß stets die gewünschte Wassermenge den weiteren Leitungsabschnitt durchströmt. Außerhalb des Betriebs des Dialysegerätes spült diese Wassermenge die sekundäre Ringleitung und einen Teil des Wassereinlaßventils. Während des Betriebs des Dialysegerätes versorgt der die Zuleitung bildende Leitungsabschnitt das Dialysegerät mit dem benötigten Wasser und kann gleichzeitig den die Rückleitung der sekundären Ringleitung bildenden weiteren Leitungsabschnitt mit Spülwasser speisen.

Nach einem alternativen Vorschlag der Erfindung zweigt der weitere Leitungsabschnitt der sekundären Ringleitung von dem ersten Leitungsabschnitt in Fließrichtung vor dem Einlaßventil ab. Die Abzweigstelle liegt zweckmäßigerweise unmittelbar vor dem Einlaßventil, so daß auch in diesem Fall praktisch der gesamte Bereich der Wasserzuführung in den Spül- bzw. Desinfektionskreislauf eingebunden ist.

Weitere Einzelheiten der Erfindung ergeben sich aus der nachfolgenden Beschreibung zweier Ausführungsformen sowie anhand der Zeichnung. Dabei zeigen auf rein schematische Weise:
- Fig. 1: eine herkömmliche Wasserversorgungseinrichtung für ein Dialysegerät;
- Fig. 2: eine erste Ausführungsform der Erfindung und
- Fig. 3: eine zweite Ausführungsform.

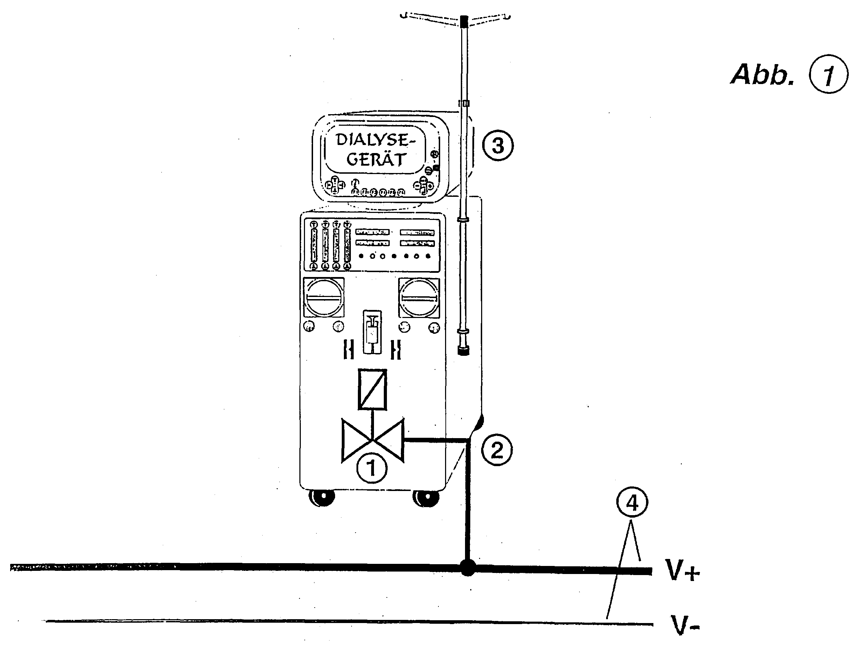

Fig. 1 zeigt rein schematisch, daß ein Wassereinlaßventil 1 eines Dialysegerätes 3 über eine Stichleitung 2 mit dem Zuleitungsabschnitt V+ mit höherem Druck einer primären Ringleitung 4 für aufbereitetes Wasser verbunden ist, neben der in der rein schematischen Zeichnung auch ein Rückleitungsabschnitt V- mit niedrigem Druck als bei V+ (in der Figur unter dem Zuleitungsabschnitt) dargestellt ist. Die Stichleitung 2 wird nur durchströmt, wenn das Dialysegerät mit aufbereitetem Wasser versorgt wird.

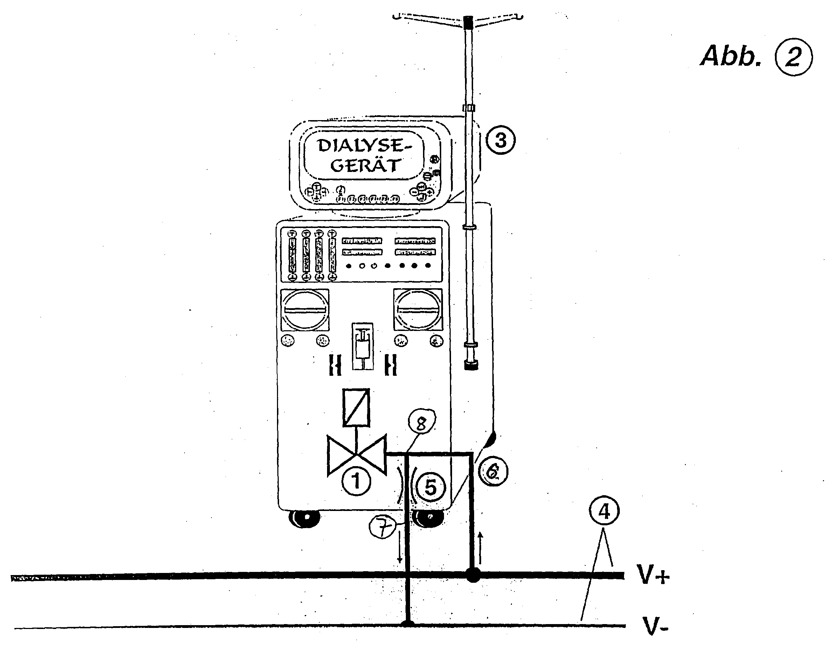

Bei der in Abbildung 2 dargestellten Ausführungsform der Erfindung ist eine sekundäre Ringleitung ausgebildet, die aus einem ersten, die Zuleitung bildenden Leitungsabschnitt 6 und einem weiteren, die Rückleitung zu der primären Ringleitung 4 bildenden Leitungsabschnitt 7 besteht. Der Leitungsabschnitt 7 enthält eine Engstelle 5, die beispielsweise durch eine Drosseleinrichtung gebildet sein kann.

Bei dieser Ausführungsform strömt ständig eine von dem Zuleitungsabschnitt der ersten Ringleitung 4 entnommene Wassermenge durch die sekundäre Ringleitung 6, 7, wobei die letztere in den Rückleitungsabschnitt der primären Ringleitung einmündet. Da die Abzweigstelle 8 dicht bei dem Wassereinlaßventil 1 liegt, wird die Ablagerung organischer Substanzen etc. in dem Wasserversorgungssystem zuverlässig verhindert. Außerdem kann die sekundäre Ringleitung 6, 7 zusammen mit der primären Ringleitung 4 desinfiziert werden.

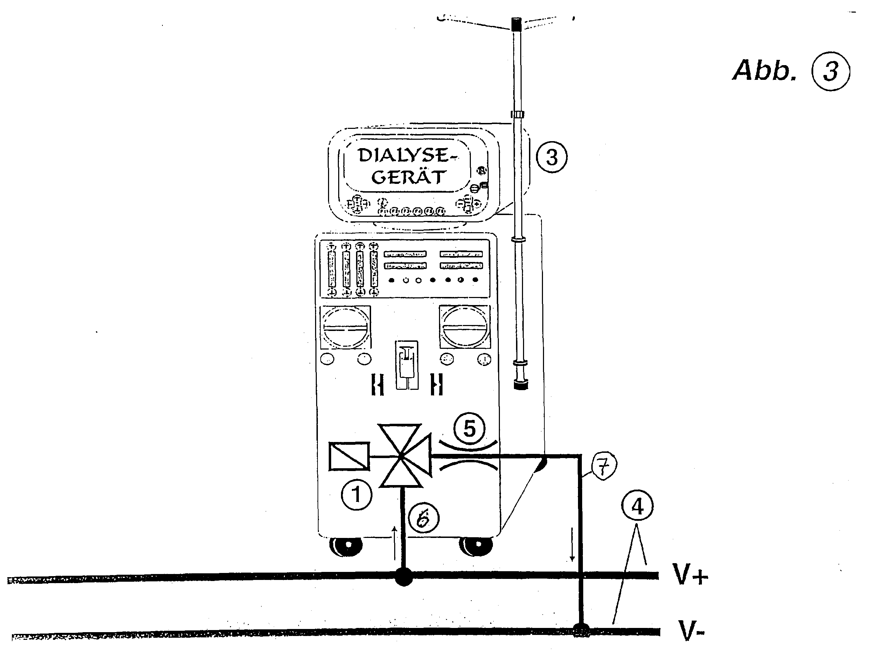

Bei der in Figur 3 dargestellten zweiten Ausführungsform der Erfindung mündet der erste Leitungsabschnitt 6 der sekundären Ringleitung in einen Anschluß des als Drei-Wege-Ventil ausgebildeten Wassereinlaßventils 1. Der weitere Leitungsabschnitt 7 mit der Engstelle 5 ist an einen anderen Anschluß des Drei-Wege-Ventils angeschlossen. Bei dieser Ausbildung ist auch das Drei-Wege-Ventil in den Spül- bzw. Desinfektionskreislauf eingebunden.

## Patentansprüche

1. Einrichtung zur Versorgung wenigstens eines, vorzugsweise mehrerer Dialysegeräte mit aufbereitetem Wasser, mit einer Ringleitung (4), von der jeweils eine Zuleitung (6) zu einem Wassereinlaßventil (1) eines Dialysegerätes (3) führt,
**dadurch gekennzeichnet**,
daß die Zuleitung ein erster Leitungsabschnitt (6) einer sekundären Ringleitung (6, 7) ist, die während und/oder außerhalb des Betriebs des Dialysegerätes (3) von dem aufbereiteten Wasser durchströmbar ist.

2. Einrichtung nach Anspruch 1,
dadurch gekennzeichnet, daß ein weiterer Leitungsabschnitt (7) der sekundären Ringleitung (6, 7) zu der primären Ringleitung (4) zurückführt.

3. Einrichtung nach Anspruch 2,
dadurch gekennzeichnet, daß der weitere Leitungsabschnitt (7) eine Engstelle (5) hat.

4. Einrichtung nach Anspruch 3,
dadurch gekennzeichnet, daß die Engstelle (5) durch eine Drosseleinrichtung gebildet ist.

5. Einrichtung nach Anspruch 4,
dadurch gekennzeichnet, daß die Drosseleinrichtung verstellbar ist.

6. Einrichtung nach Anspruch 2,
dadurch gekennzeichnet, daß der weitere Leitungsabschnitt (7) einen kleineren Innendurchmesser als der erste Leitungsabschnitt (6) hat.

7. Einrichtung nach Anspruch 2,
dadurch gekennzeichnet, daß der weitere Leitungsabschnitt (7) an das Wassereinlaßventil (1) des Dialysegerätes (3) angeschlossen ist, welches durch ein Drei-Wege-Ventil gebildet ist.

8. Einrichtung nach einem der Ansprüche 1 bis 6,
dadurch gekennzeichnet, daß der weitere Leitungsabschnitt (7) von dem ersten Leitungsabschnitt (6) in Fließrichtung vor dem Einlaßventil (1) abzweigt.

9. Einrichtung nach Anspruch 2,
dadurch gekennzeichnet, daß der weitere Leitungsabschnitt an einen Überströmungskanal eines Zwei-Wege-Ventils angeschlossen ist.
